# EUROPEAN PATENT SPECIFICATION

(11) **EP 3 786 638 B1**
(45) Date of publication and mention of the grant of the patent: **02.08.2023**
(21) Application number: 19793642.0
(22) Date of filing: 24.04.2019
(51) Int. Cl.: G01N 21/31, G01N 21/82, G01N 33/72, B01L 3/00, G01N 1/00, G01N 33/574, G01N 33/543, A61B 10/00

(54) **STOOL SPECIMEN EXAMINING DEVICE, AND STOOL SPECIMEN EXAMINING METHOD**
VORRICHTUNG ZUR UNTERSUCHUNG VON STUHLPROBEN UND VERFAHREN ZUR UNTERSUCHUNG VON STUHLPROBEN
DISPOSITIF D'EXAMEN D'ÉCHANTILLON DE SELLES, ET PROCÉDÉ D'EXAMEN D'ÉCHANTILLON DE SELLES

(30) Priority: 26.04.2018 JP 2018085688
(43) Date of publication of application: 03.03.2021
(73) Proprietor: EIKEN KAGAKU KABUSHIKI KAISHA, Tokyo 110-8408 (JP)
(72) Inventor: YAMAOKA, Toshihiko, Shimotsuga-gun, Tochigi 329-0114 (JP); YAMAMOTO, Ayaka, Shimotsuga-gun, Tochigi 329-0114 (JP); YUI, Megumi, Shimotsuga-gun, Tochigi 329-0114 (JP); MAKINODAN, Mitsuru, Shimotsuga-gun, Tochigi 329-0114 (JP)
(74) Representative: Altmann Stößel Dick Patentanwälte PartG mbB
(86) International application number: PCT/JP2019/017506
(87) International publication number: WO 2019/208659

(56) References cited:
- WO-A1-01/61343
- JP-A- H10 311 828
- JP-A- 2005 181 158
- US-A1- 2006 216 830
- US-A1- 2006 216 830

## Description

### [Technical Field]

The present invention relates to a technique of stool specimen test. In particular, the present invention relates to a stool specimen test device and to a method for stool specimen test.

### [Background Art]

Stool is a biological sample. In particular, it is an important specimen from which information inside the gastrointestinal tract can be obtained. A major portion of stool is made up of moisture, and the rest thereof is made up of dead intestinal wall cells, dead enteric bacteria, and food debris. Useful information on digestive system diseases can be obtained from stool, and such information is beneficial for disease diagnosis.

Large bowel cancer that is increasing in recent years occasionally causes a microscopic amount of blood to adhere the stool surface when the stool passes through the large bowel at an early stage with no subjective symptoms. Such a microscopic amount of blood cannot be visually detected and thus is referred to as the "fecal occult blood." The fecal occult blood test is intended to detect polyps of the large bowel or early-stage large bowel cancer by detecting the fecal occult blood. The fecal occult blood test can be performed in a relatively cost-effective manner with a reduced burden on a subject. Thus, it is extensively practiced for "large bowel cancer screening."

As methods for fecal occult blood test, the Guaiac test or the orthotolidine test involving the use of biochemical reactions had been performed in the past; however, such test methods are not substantially performed because of low specificity. A new mainstream is detection of a fecal occult blood marker by an immunological method. While a representative fecal occult blood marker is hemoglobin, blood-derived substances, such as calprotectin, transferrin, and a hemoglobin-haptoglobin composite, are also used as the fecal occult blood markers.

The fecal occult blood test is intended to detect the bleeding in the gastrointestinal tract, and it is thus not always specific for large bowel cancer. Accordingly, a marker for large bowel cancer in the stool has been continuously searched. For example, Patent Document 1 reports a biomarker for large bowel cancer on the basis of the results of mass analysis of a stool specimen. Also, Patent Document 2 reports methylation of a cancer-cell-specific gene contained in the stool.

A process example of the fecal occult blood test is provided below.
1) A subject of the fecal occult blood test collects a stool specimen in a dedicated-purpose sampling container at home.
2) A subject submits a sampling container in which the collected stool specimen is accommodated to the test institution.
3) At the test institution, an examiner mounts the sampling container on the test device and performs the fecal occult blood test (the test may be performed manually).

When the result of the fecal occult blood test is positive, the presence of polyps of the large bowel or large bowel cancer is suspected. In such a case, a detailed examination, such as large bowel endoscopy, is performed.

Since a stool specimen is paste, it is more difficult to collect a given amount of a stool specimen, compared to a liquid biological sample, such as blood or urine. While reagent manufacturers that sell reagents for the fecal occult blood test have attempted development of sampling containers that can constantly collect a given amount of a stool specimen, the issue of an excess amount or a lack of the specimens remains unresolved.

A stool specimen is often collected by a subject at home. Accordingly, the amount thereof collected often varies depending on a procedure taken by the subject. Because of a misconception or an inadequate procedure taken by the subject, the amount of the stool specimen may occasionally be excessively small, or the sampling container without the collected stool sample may occasionally be submitted to the test institution.

Even when there is no collected stool sample in the container, it is difficult to detect the absence of a stool sample from the outside of the container for the following reasons. That is, the amount of a stool sample necessary for the fecal occult blood test is very small, and the collected stool specimen is suspended and dispersed in the sampling buffer in the container. Even if an adequate amount of a stool sample is collected, accordingly, it is impossible to visually detect the presence or absence of the stool specimen from the outside of the container. When the specimen containing no collected stool sample is subjected to the fecal occult blood test, the test result always becomes negative, and polyps of the large bowel or large bowel cancer may accordingly be not detected.

Patent Document 3 discloses a method comprising measuring the absorbance of indigenous substances in the stool to be tested or a stool suspension to correct the amount of stool when measuring hemoglobin and/or transferrin as a fecal occult blood marker. According to Patent Document 3, the concentration of a fecal occult blood marker can be measured with high accuracy; however, Patent Document 3 does not disclose a method of determining as to whether or not a fecal occult blood marker was detected or the specimen contained the collected stool sample when the specimens was negative for the fecal occult blood test.

Patent Document 4 discloses a feces sampling container for use in assaying feces as a specimen, and a method and analyzer for assaying feces.

### [Prior Art Documents]

### [Patent Documents]

[Patent Document 1] WO 2013/162368 A1
[Patent Document 2] WO 2017/043497 A1
[Patent Document 3] WO 01/61343 A1
[Patent Document 4] US 2006/0216830 A1

### [Summary of the Invention]

### [Objects to Be Attained by the Invention]

It is an object of the present invention to detect the specimens without the collected stool sample in the stool specimen test to thereby avoid missing of the detection of an analyte contained in a stool specimen and improve the accuracy of the stool specimen test.

### [Means for Attaining the Objects]

The object of present invention is attained by a stool specimen test device as defined in independent claim 1. The object of present invention is also attained by a method for stool specimen test as defined in appended claim 11. Particular embodiments of the invention are defined in the appended dependent claims.

### [Advantageous Effects of the Invention]

According to the present invention, a specimen without the collected stool sample is detected in the stool specimen test, so as to suppress missing of the detection of an analyte contained in a stool specimen and improve the accuracy of the stool specimen test.

### [Brief Description of the Drawings]

Fig. 1 schematically shows a configuration example of an analysis mechanism based on a squeeze-up system.
Fig. 2 shows a functional block diagram indicating a configuration example of a fecal occult blood test device comprising the sampling verification mechanism according to an embodiment of the present invention.
Fig. 3 shows a flowchart indicating a process example of the fecal occult blood test using the fecal occult blood test device comprising the sampling verification mechanism shown in Fig. 2.
Fig. 4 shows a sequence diagram indicating the process of treatment of Fig. 3 in chronological order.
Fig. 5 shows an example of an absorbance spectrum of a stool suspension.

### [Embodiments of the Invention]

Hereafter, the stool specimen test device according to an embodiment of the present invention is described in detail with reference to the drawings. The stool specimen test device according to the present embodiment comprises a sampling verification mechanism that detects a specimen without the collected stool sample.

A sample that is tested in the present invention contains a solution including a buffer in which the collected stool sample is suspended. In addition, such sample preferably contains a stool specimen.

The stool specimen test device of the present invention is useful for detecting a specimen without the collected stool sample, which does not substantially contain the collected stool sample. The wording "without the collected stool sample" used herein refers to a situation in which a sampling container does not substantially contain the collected stool. When a specimen "does not substantially contain the collected stool," as described below, the specimen contains a stool sample in a small amount that exhibits the absorbance equivalent to or lower than the threshold determined to evaluate whether or not the specimen contains the collected stool sample, or the specimen does not contain any stool sample. As a possible cause of "the specimen without the collected stool sample," a subject could not collect a stool specimen due to an insufficient procedure in spite of his/her intention, a subject may have collected a very small amount of stool sample, or a subject may have erroneously submitted a sampling container without the collected stool sample because he/she did not perform stool sampling.

### (Entire configuration)

The stool specimen test device of the present invention comprises the features defined in appended claim 1, including sample suction section, a sampling verification section, and a sample test section. According to an embodiment of the present invention, in addition, a stool specimen test device comprises a control section. When a large number of specimens are handled, in addition, the device comprises a transport section that transports a sampling container or a specimen rack that accommodates a sampling container, according to need.

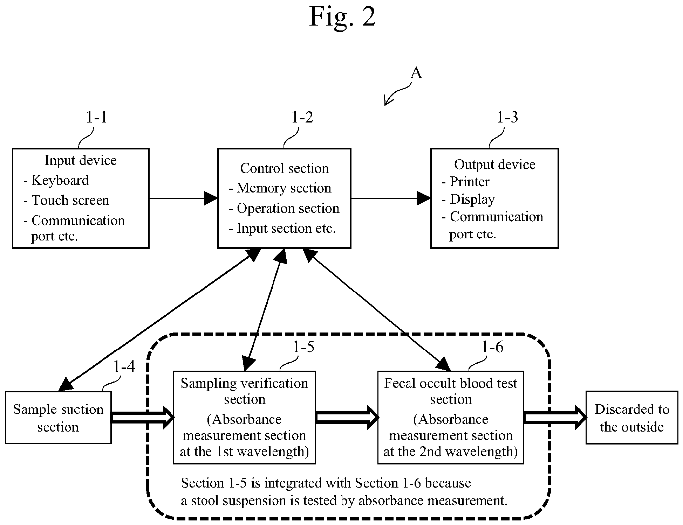

Fig. 2 shows a functional block diagram indicating a configuration example of a sampling verification system A comprising the stool specimen test device. The sampling verification system A comprises a input device 1-1, a control section 1-2, an output device 1-3, a sample suction section 1-4, a sampling verification section 1-5, and a fecal occult blood test section 1-6. Specific examples of each functional section are shown in Fig. 2.

In Fig. 2, an arrow indicates a flow of an input/output signal, and an outlined arrow indicates a flow of a stool suspension.

### (Sample suction port)

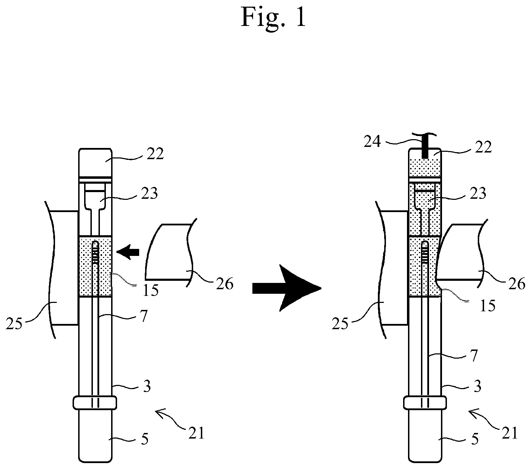

At the sample suction section 1-4, a sample is collected from the sampling container and delivered to the sampling verification section 1-5. With reference to a case in which a sample contains a stool specimen, a process of sample collection is shown in Fig. 1. A sampling container 21 comprises a container body 3, a sample cup 22, and a container lid 5 provided to fit the container body 3. The container lid 5 comprises a sample collection rod 7 for collecting a stool specimen. The container body 3 accommodates a solution including a buffer in which the collected stool sample is suspended. The sampling container 21 that contains a stool specimen collected by the subject with the use of the sample collection rod 7 and a solution in which the collected stool sample is suspended (hereafter, referred to as a "stool suspension" 15) is mounted on the sample suction port of the stool specimen test device in a manner such that the sample cup 22 is positioned on the top.

A lateral side of the sampling container 21 is pushed at a push section 26 to push up the liquid level of the stool suspension inside the sampling container, the stool suspension is filtered through the filter section 23 from the inside of the sampling container body 3, and the stool suspension is retained in the sample cup 22 (a squeeze-up system). A sample nozzle 24 is inserted into a sample suction port of the sample cup 22 to suction the stool suspension retained in the sample cup 22. The suctioned stool suspension is then delivered to the sampling verification section and the sample test section for measurement. A numerical reference 25 indicates a holding section that can hold the sampling container body 3. A specimen without the collected stool sample is treated in the same manner.

### (Sampling verification section)

At a sampling verification section 1-5, the absorbance of the sample suctioned at the sample suction section 1-4 is measured. The measured absorbance value is transferred to the control section 1-2. At the sampling verification section 1-5, whether or not the sample substantially contains a stool specimen; i.e., the specimen is without the collected stool sample, is determined. Since the stool suspension is tested by measuring the absorbance, the sampling verification section 1-5 is preferably integrated with the fecal occult blood test section 1-6.

As the indicators of sampling verification, for example, the concentration of indigenous substances in stool, such as inorganic phosphorus, calcium, magnesium, alkaline phosphatase, and amylase, and the absorbance of the sample can be employed. Measurement of the absorbance of the stool suspension is particularly preferable since no reagents or reactions are necessary for sampling verification, and the stool suspension subjected to measurement of the absorbance can be subjected to the subsequent stool specimen test without any treatment.

### (Measurement of absorbance)

For sampling verification, the absorbance can be measured at any wavelength within the range of 300 nm to 510 nm without particular limitation, but a range of 300 nm to 450 nm is preferable since a difference in the absorbance between the sample buffer and the stool suspension is increased. For sampling verification, the absorbance is preferably measured at the wavelength selected from among 300 nm, 340 nm, 400 nm to 415 nm, 445 nm to 455 nm, and 480 nm to 505 nm. This enables the use of an optical system of a common chemistry analyzer for sampling verification and cost-reduction of the device.

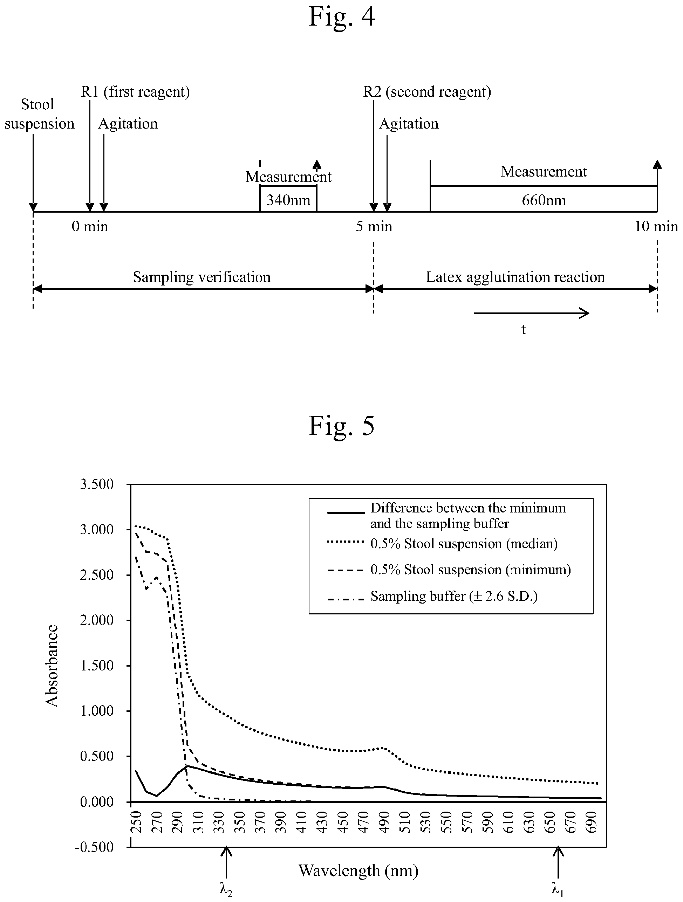

Fig. 5 shows the absorbance of 31 normal stool specimens in a 0.5% stool suspension and in a solution in which the collected stool sample is suspended, the horizontal axis indicates the measurement wavelength, and the vertical axis indicates the absorbance. In Fig. 5, a dash-dot-dash line represents the absorbance in a solution in which the collected stool sample is suspended (mean + 2.6 S.D.), a dot line represents the absorbance of 31 stool suspension specimens (the median), and a broken line represents the absorbance of 31 stool suspension specimens (the minimum). A solid line plots a difference between the absorbance in the solution in which the collected stool sample is suspended and the absorbance in the 0.5% stool suspension (the minimum).

A plot indicated by a dash-dot-dash line represents the mean + 2.6 S.D. of the absorbance of a plurality of plots. Thus, the absorbance of the solution in which the collected stool sample is suspended would not be substantially higher than the plot indicated by the dash-dot-dash line (statistical probability: 0.5%). The plot indicated by the solid line demonstrates a sufficient difference between the absorbance of the solution in which the collected stool sample is suspended and the absorbance of the stool suspension (the minimum) in the wavelength range of 300 nm to 510 nm. If the threshold is determined on the basis of the absorbance of the solution in which the collected stool sample is suspended measured in advance, accordingly, a specimen exhibiting the absorbance equivalent to or lower than the threshold can be detected as a specimen that does not contain any stool sample or a specimen that contains a very small amount of the collected stool sample; i.e., the specimen "without the collected stool sample" according to the present invention.

A container used for measurement of the absorbance is preferably a disposable plastic cell from the viewpoint of contamination prevention and the cost. A transmission wavelength of a polystyrene cell is from 320 nm to 800 nm, and that of polymethyl methacrylate resin is from 285 nm to 800 nm. An adequate cell may be selected in accordance with the measurement wavelength.

When the amount of the sample solution is not sufficient for measurement of the absorbance, a buffer or other substances can be added while refraining from influencing the measurement.

### (Verification and judgment of stool sampling)

The absorbance measured at the sampling verification section is transferred to the control section to determine whether or not stool sampling had been performed. When the absorbance of the sample exceeds the threshold, the sample is determined to contain a stool specimen, and the sample is then subjected to the subsequent stool specimen test.

When the absorbance of the sample is equivalent to or lower than the threshold, the sample is determined to be without the collected stool, and at least one of the treatments described below is then executed.
(1) An alarm is set off.
(2) The test is suspended.
(3) The information of the uncollected stool sample is printed using a printer.
(4) The information indicating the specimen without the collected stool sample is shown on a display screen.
(5) The information indicating the specimen without the collected stool sample is transmitted to an external device.

When a fecal occult blood marker is detected in large bowel cancer screening, many specimens are mounted on the device at the beginning, and there would be no persons around the device until the completion of the measurement. Thus, it is preferable that at least one of the treatments (3) to (5) be executed. The treatments (3) to (5) may be successively executed. Alternatively, the data concerning the specimens without the collected stool sample may be stored in the memory section attached to the control section and collectively outputted after the completion of the measurement.

### (Sample test section)

At the sample test section, an analyte contained in a stool specimen, such as a marker or pathogen of the digestive system disease, is detected. A representative example is detection of a fecal occult blood marker, which serves as the indicator for large bowel cancer. Examples of other items that can be tested include the following.

### (1) Tumor markers, such as M2PK, IGFBP2, and EpCAM

A marker for large bowel cancer that is present in the stool may be detected by an immunological method, so that specificity/sensitivity of large bowel cancer detection can be enhanced in combination with the results of the fecal occult blood marker test.

### (2) Gene markers, such as p53, K-ras, and Twist1

Nucleic acids are extracted from a stool suspension and analyzed to detect abnormal gene expression, mutation, or methylation specifically observed in large bowel cancer. With the use of the results of analysis in combination with the results of the fecal occult blood marker test, specificity/sensitivity of large bowel cancer detection can be enhanced.

### (3) Detection of causative agent of food poisoning

Enterohemorrhagic E. coli, a toxin thereof, Norwalk parvovirus, rotavirus, and the like are detected to identify the cause of food poisoning.

### (4) Helicobacter pylori antigen

Helicobacter pylori that increases a risk of chronic gastritis, gastric ulcer, or gastric cancer is detected to determine the occurrence of Helicobacter pylori infection.

With the device and method of the present invention, a specimen without the collected stool sample can be detected in the stool specimen test to avoid missing in the stool specimen test and improve the accuracy of the stool specimen test. According to an embodiment of the present invention, missing of the detection of the fecal occult blood can be avoided, and the accuracy of the fecal occult blood test can be improved.

### (Method of measurement)

Methods of measurement performed at the sample test section are roughly classified into the method of immunological measurement and the method of molecular biological measurement.

### (Method of immunological measurement)

When a target of measurement is, for example, a protein, glycoprotein, or polysaccharide, the method of immunological measurement is employed. The method of immunological measurement involves the use of specific binding between an antigen and an antibody, and various methods are known. Representative examples include latex agglutination turbidimetric immunoassay (LATIA) and enzyme-linked immunosorbent assay (EIA and ELISA). According to the method of immunological measurement, a fecal occult blood marker, a tumor marker, and a pathogen-derived antigen can be detected.

### (Method of molecular biological measurement)

When a target of measurement is a gene or genetic mutation, the method of molecular biological measurement is employed. The method of molecular biological measurement involves the use of properties such that a single-stranded nucleic acid (DNA or RNA) specifically binds to another single-stranded nucleic acid having a nucleotide sequence complementary to that of the former single-stranded nucleic acid. With the use of such properties, a nucleotide sequence (a probe) complementary to the target of measurement is prepared and a gene expressed specifically in a cancer cell or a pathogen-specific gene is detected. When genetic mutation is to be detected, a probe that specifically binds to the site of mutation is used. As methods for detection of cancer-cell-specific methylation, a method involving the use of nucleotide substitution via bisulfite treatment and a method involving the use of a methylation-specific restriction enzyme are known.

When performing the method of molecular biological measurement, a target gene can be amplified in advance. Gene amplification can be performed in accordance with a conventional gene amplification method, such as PCR, SDA, NASBA, or LAMP. When a target of measurement is a bacterium or virus, a target-specific gene may be amplified, and the target can be detected based on the presence or absence of the amplified product.

### (Configuration of sample test section)

The sample test section comprises a reaction container that accommodates a sample containing a stool specimen, a reagent supply section that supplies various reagents to the reaction container, and a detection section that detects an analyte after the reaction with a reagent. The reaction container can also serve as a measurement section. When an analyte is detected via optical measurement, in addition, the reaction container can be integrated with a measurement container of the judgment section as to a lack of the analyte. The sample test section may comprise an agitation mechanism, a temperature control mechanism capable of heating and cooling, and the like in accordance with the method of measurement.

### (Method for measurement of fecal occult blood)

The fecal occult blood test for which the present invention is particularly useful is performed in the manner described below.

Examples of the fecal occult blood markers to be measured in large bowel cancer screening include hemoglobin, calprotectin, transferrin, and a hemoglobin-haptoglobin composite. All such the fecal occult blood markers are blood-derived protein components.

A method for measurement of a fecal occult blood marker is not particularly limited, provided that it is a method of immunological measurement. Latex agglutination turbidimetric immunoassay (LATIA) is suitable because it does not require detection sensitivity or B/F separation. According to LATIA, a fecal occult blood marker is detected by measuring the absorbance. Thus, the reaction container in the sample test section also serves as a container for measurement of the absorbance, and it further serves as a container for measurement of the absorbance at the sampling verification section.

Fig. 2 shows a functional block diagram indicating a configuration example of a fecal occult blood test device comprising the sampling verification mechanism according to an embodiment of the present invention. While an example in which the fecal occult blood is detected by measuring the hemoglobin concentration is described herein, the target of detection is not limited to hemoglobin.

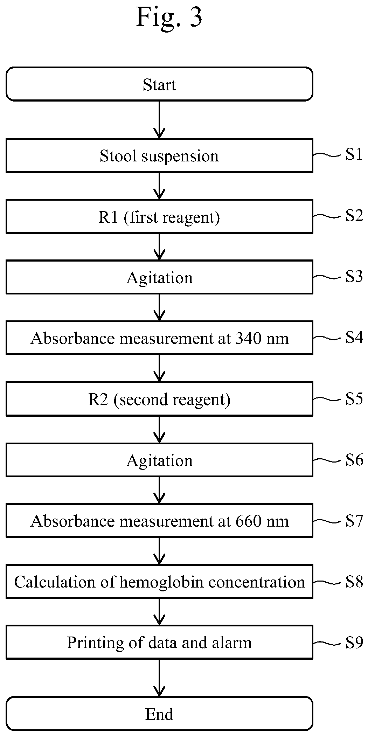

Fig. 3 shows a flowchart indicating a process example of the fecal occult blood test using the fecal occult blood test device comprising the sampling verification mechanism shown in Fig. 2. Fig. 4 shows a sequence diagram indicating the process example shown in Fig. 3 in chronological order.

Hereafter, measurement of a fecal occult blood marker is described in detail with reference to Fig. 3.

In Step S1, the stool suspension collected from the sample cup of the sampling container is transported to the judgment section for the sampling verification that also serves as the fecal occult blood test section, and the stool suspension is dispensed into measurement cuvettes . In Step S2, the first reagent is f dispensed into measurement cuvettes. In Step S3, the sample is mixed with the first reagent with agitation. Within 5 minutes thereafter, for example, light at the first wavelength of 340 nm is applied to measure the absorbance for judgment of a lack of the analyte in Step S4.

The first reagent contains a component that increases the amount of the sample for measurement of the absorbance and also neutralizes an inhibitor in the antigen-antibody reaction that may be contained in the sample. During such neutralizing reaction; i.e., during a period from the addition of the first reagent to the addition of the second reagent, the absorbance may be measured at any timing.

The absorbance measured in the step of verification of stool-sampling is transferred to the control section described below to evaluate whether or not the sample contains the collected stool. When the sample is evaluated to contain the collected stool, the subsequent step of measurement of a fecal occult blood marker is performed. When the sample is evaluated to contain no collected stool, the subsequent step of measurement of a fecal occult blood marker is performed with the information of the uncollected stool sample.

In Step S5, subsequently, the second reagent containing antibody-sensitized latex particles is dispensed into measurement cuvettes, and the latex agglutination reaction is then performed with agitation in Step S6.

In Step S7, subsequently, the absorbance of the reaction solution is measured at the second wavelength. The second wavelength is, for example, 660 nm. The wavelength at which agglutination of the reaction solution is measured is not limited to a single wavelength. In addition to the primary wavelength, the absorbance may be simultaneously measured at the secondary wavelength. The assay accuracy can be thus improved.

The latex agglutination reaction may be examined by an end-point assay in which the absorbance is measured after a given reaction period. Alternatively, a rate assay may be performed. In the rate assay, the absorbance is first measured immediately after the initiation of the reaction, measurement is performed a plurality of times with the elapse of time, and a change in the absorbance is then calculated.

The measured absorbance value of the latex agglutination reaction is transferred to the control section and converted to the measured value of a fecal occult blood marker. The measured value of a fecal occult blood marker is printed using a printer, shown on a display screen, or subjected to necessary processing (Step S8: hemoglobin concentration is calculated). Thus, measurement on an analyte is completed (Step S9: e.g., the data is obtained and the alarm is printed when hemoglobin is detected), and another analyte is then subjected to measurement.

The number of types of the fecal occult blood markers measured at the fecal occult blood measurement section is not limited to 1, and a plurality of markers can be measured. For example, it is possible to measure hemoglobin, collect another stool suspension from the same sampling container, and then measure calprotectin. A tumor marker that can be detected in the stool and measured via latex agglutination turbidimetric immunoassay can also be employed as the item of measurement.

When the fecal occult blood test performed after sampling verification is also performed by measuring the absorbance, the measurement wavelength may overlap with the measurement wavelength employed for sampling verification.

The sampling verification section and the sample test section can be configured as described below.
(1) The sampling verification section is provided independently of the sample test section, samples are supplied thereto, and measurement is performed. This configuration is suitable when a stool specimen is tested by a method other than measurement of the absorbance. For sampling verification, a reagent that may affect the target substance or the reaction of the stool specimen test may be added.
(2) The sampling verification section is integrated with the sample test section, and the sample subjected to sampling verification is subjected to the stool specimen test without further processing. This configuration is preferable when stool sampling is verified by measuring the absorbance. When a reagent is added at the time of sampling verification, it is necessary to suppress the influence imposed on the target substance or the reaction of the subsequent stool specimen test. The absorbance can be measured using a flow cell which is a flow path toward the sample test section, or the measurement container (cell) used for sampling verification can also be used as a reaction/measurement container for the subsequent stool specimen test. Because of such configuration, the amount of the sample necessary for sampling verification and test can be reduced.

According to the method described above, the specimen evaluated to be without the collected stool sample is subjected to the subsequent stool specimen test. When the specimen is evaluated to be without the collected stool sample, alternatively, the stool specimen test may not be performed. Also, the stool specimen may be first tested, and stool sampling may then be verified.

### (Control section)

As shown in Fig. 2, the control section 1-2 comprises an operation section, a memory section, and the like, and an input section that imports the measured absorbance value, the input device 1-1, such as a keyboard and a touch screen, and the output device 1-3, such as a display screen or a printer, are connected to the control section 1-2. In order to perform data processing or observation of the operation status of remote maintenance or the test device using an external device, the control section 1-2 may be provided with various communication ports, such as RS-232C, LAN, or USB. The control section 1-2 may be integrated into the stool specimen test device of the present invention, or the control section 1-2 may be independently provided outside the stool specimen test device. In the case of the latter, a control section may be able to control a plurality of stool specimen test devices.

The control section 1-2 may be configured to verify stool sampling based on the measured absorbance value at the first wavelength. In addition, the control section 1-2 may be configured to calculate the concentration of a fecal occult blood marker based on the measured absorbance value at the second wavelength, control the operation of the entire test device, or deal with various errors.

### [Industrial Applicability]

When the stool specimen test device yields negative results concerning the occult blood or the tumor marker in the stool specimen, the present invention can induce re-examination with reference to the information indicating the absence of the collected stool sample and prevent missing of digestive system diseases, including large bowel cancer.

### [Description of References]

A: Sampling verification system
1-1: Input device
1-2: Control section
1-3: Output device
1-4: Sample suction section
1-5: Sampling verification section (absorbance measurement section)
1-6: Fecal occult blood test section (absorbance measurement section)
3: Container body
5: Container lid
7: Sample collection rod
15: Stool suspension
21: sampling container
22: Sample cup
23: Filter section
26: Push section
24: Sample nozzle
25: Holding section

## Claims

1. A stool specimen test device (A) comprising a sampling verification section (1-5), a sample suction section (1-4) for collecting a sample from a sampling container (21) and delivering the collected sample to the sampling verification section (1-5), the sampling verification section configured to detect a sample without a collected stool specimen based on a comparison result of the absorbance of the sample measured at a first wavelength and a threshold determined in advance, wherein the threshold is determined based on the absorbance of a solution in which the collected stool specimen is suspended measured at the first wavelength, and a sample test section (1-6) for detecting fecal occult blood contained in the sample,
wherein the sample contains a sampling buffer and can further contain a stool specimen.

2. The stool specimen test device according to claim 1, wherein the sample without the collected stool specimen exhibits the absorbance at a level equivalent to or lower than the threshold determined in advance and the threshold is determined based on the absorbance of a solution in which the collected stool specimen is suspended.

3. The stool specimen test device according to claim 1 or 2, wherein the sample test section comprises an absorbance measurement section configured to measure at a second wavelength.

4. The stool specimen test device according to any one of claims 1 to 3, wherein the first wavelength is a wavelength selected from the wavelength range from 300 nm to 510 nm.

5. The stool specimen test device according to any one of claims 1 to 3, wherein the first wavelength is a wavelength selected from the wavelength range from 300 nm to 450 nm.

6. The stool specimen test device according to any one of claims 1 to 3, wherein the first wavelength is a wavelength selected from the wavelength range from 295 nm to 305 nm, a wavelength selected from the wavelength range from 335 nm to 345 nm, a wavelength selected from the wavelength range from 375 nm to 385 nm, a wavelength selected from the wavelength range from 400 nm to 415 nm, a wavelength selected from the wavelength range from 445 nm to 455 nm, or a wavelength selected from the wavelength range from 480 nm to 505 nm.

7. The stool specimen test device according to any one of claims 1 to 6, wherein the sample test section (1-6) is configured to perform an immunological assay.

8. The stool specimen test device according to claim 7, wherein the sample test section (1-5) is configured to perform the immunological assay by an agglutination assay involving the use of insoluble carrier particles.

9. The stool specimen test device according to any one of claims 1 to 8, wherein the sample containing the sampling buffer and the stool specimen is a stool suspension (15), and wherein the sample test section (1-5) is configured to detect a fecal occult blood marker and/or a tumor marker in the stool suspension (15).

10. The stool specimen test device according to any one of claims 3, wherein the sampling verification section (1-5) is configured to measure the absorbance at the first wavelength is also configured to serve as the absorbance measurement section at the second wavelength.

11. A method for stool specimen test comprising:
a step of measuring an absorbance value of a sample at a first wavelength, wherein the sample has been collected from a sampling container (21);
a step of sampling verification for detecting a sample without a collected stool specimen based on a comparison result of the measured absorbance value and a threshold determined in advance, wherein the threshold is determined based on the absorbance of a solution in which the collected stool specimen is suspended measured at the first wavelength; and
a step of sample test for detecting fecal occult blood contained in the sample,
wherein the sample contains a sampling buffer and can further contain a stool specimen.

12. The method for stool specimen test according to claim 11, wherein the sample without the collected stool specimen exhibits the absorbance at a level equivalent to or lower than the threshold determined in advance and the threshold is determined based on the absorbance of a solution in which the collected stool specimen is suspended.

13. The method for stool specimen test according to claim 11 or 12, wherein the first wavelength is a wavelength selected from the wavelength range from 300 nm to 510 nm.

14. The method for stool specimen test according to claim 11 or 12, wherein the first wavelength is a wavelength selected from the wavelength range from 300 nm to 450 nm.

15. The method for stool specimen test according to claim 11 or 12, wherein the first wavelength is a wavelength selected from the wavelength range from 295 nm to 305 nm, a wavelength selected from the wavelength range from 335 nm to 345 nm, a wavelength selected from the wavelength range from 375 nm to 385 nm, a wavelength selected from the wavelength range from 400 nm to 415 nm, a wavelength selected from the wavelength range from 445 nm to 455 nm, or a wavelength selected from the wavelength range from 480 nm to 505 nm.

16. The method for stool specimen test according to any one of claims 11 to 15, wherein the step of sample test performs measurement of the absorbance at a second wavelength that is different from the first wavelength.

17. The method for stool specimen test according to any one of claims 11 to 16, wherein in the step of sample test the sample is tested by an immunological assay method.

18. The method for stool specimen test according to claim 17, wherein the immunological assay is an agglutination assay involving the use of insoluble carrier particles.

19. The method for stool specimen test according to any one of claims 11 to 18, wherein the step of sample test comprises detecting a fecal occult blood marker and/or a tumor marker in the sample.

## Patentansprüche

1. Vorrichtung (A) zum Testen von Stuhlproben, umfassend einen Probenahmeverifizierungsabschnitt (1-5), einen Probensaugabschnitt (1-4) zum Sammeln einer Probe aus einem Probenahmebehälter (21) und Abgeben der gesammelten Probe an den Probenahmeverifizierungsabschnitt (1-5), wobei der Probenahmeverifizierungsabschnitt ausgestaltet ist, um eine Probe ohne eine gesammelte Stuhlprobe basierend auf einem Vergleichsergebnis der Extinktion der Probe, die bei einer ersten Wellenlänge gemessen wurde, und einem vorab bestimmten Schwellenwert zu detektieren, wobei der Schwellenwert basierend auf der Extinktion einer Lösung, in der die gesammelte Stuhlprobe suspendiert ist, gemessen bei der ersten Wellenlänge, bestimmt wird, und einen Probentestabschnitt (1-6) zum Detektieren von fäkalem okkultem Blut, das in der Probe enthalten ist,
wobei die Probe einen Probenahmepuffer enthält und des Weiteren eine Stuhlprobe enthalten kann.

2. Vorrichtung zum Testen von Stuhlproben nach Anspruch 1, wobei die Probe ohne die gesammelte Stuhlprobe die Extinktion bei einem Niveau zeigt, das gleich dem vorab bestimmten Schwellenwert ist oder darunter liegt, und der Schwellenwert basierend auf der Extinktion einer Lösung bestimmt wird, in welcher die gesammelte Stuhlprobe suspendiert ist.

3. Vorrichtung zum Testen von Stuhlproben nach Anspruch 1 oder 2, wobei der Probentestabschnitt einen Extinktionsmessabschnitt umfasst, der ausgestaltet ist, um bei einer zweiten Wellenlänge zu messen.

4. Vorrichtung zum Testen von Stuhlproben nach einem der Ansprüche 1 bis 3, wobei die erste Wellenlänge eine Wellenlänge ausgewählt aus dem Wellenlängenbereich von 300 nm bis 510 nm ist.

5. Vorrichtung zum Testen von Stuhlproben nach einem der Ansprüche 1 bis 3, wobei die erste Wellenlänge eine Wellenlänge ausgewählt aus dem Wellenlängenbereich von 300 nm bis 450 nm ist.

6. Vorrichtung zum Testen von Stuhlproben nach einem der Ansprüche 1 bis 3, wobei die erste Wellenlänge eine Wellenlänge ausgewählt aus dem Wellenlängenbereich von 295 nm bis 305 nm, eine Wellenlänge ausgewählt aus dem Wellenlängenbereich von 335 nm bis 345 nm, eine Wellenlänge ausgewählt aus dem Wellenlängenbereich von 375 nm bis 385 nm, eine Wellenlänge ausgewählt aus dem Wellenlängenbereich von 400 nm bis 415 nm, eine Wellenlänge ausgewählt aus dem Wellenlängenbereich von 445 nm bis 455 nm oder eine Wellenlänge ausgewählt aus dem Wellenlängenbereich von 480 nm bis 505 nm ist.

7. Vorrichtung zum Testen von Stuhlproben nach einem der Ansprüche 1 bis 6, wobei der Probentestabschnitt (1-6) ausgestaltet ist, um einen immunologischen Assay durchzuführen.

8. Vorrichtung zum Testen von Stuhlproben nach Anspruch 7, wobei der Probentestabschnitt (1-5) ausgestaltet ist, um den immunologischen Assay mittels eines Agglutinierungs-Assays durchzuführen, der die Verwendung von unlöslichen Trägerpartikeln beinhaltet.

9. Vorrichtung zum Testen von Stuhlproben nach einem der Ansprüche 1 bis 8, wobei die Probe, welche den Probenahmepuffer und die Stuhlprobe enthält, eine Stuhlsuspension (15) ist, und wobei der Probentestabschnitt (1-5) ausgestaltet ist, um einen Marker für fäkales okkultes Blut und/oder einen Tumormarker in der Stuhlsuspension (15) zu detektieren.

10. Vorrichtung zum Testen von Stuhlproben nach einem der Ansprüche 3, wobei der Probenahmeverifizierungsabschnitt (1-5) ausgestaltet ist, um die Extinktion bei der ersten Wellenlänge zu messen, auch ausgestaltet ist, um als Extinktionsmessabschnitt bei der zweiten Wellenlänge zu dienen.

11. Verfahren zum Testen von Stuhlproben, umfassend:
einen Schritt des Messens eines Extinktionswerts einer Probe bei einer ersten Wellenlänge, wobei die Probe aus einem Probenahmebehälter (21) gesammelt worden ist;
einen Schritt der Probenahmeverifizierung zum Detektieren einer Probe ohne eine gesammelte Stuhlprobe basierend auf einem Vergleichsergebnis des gemessenen Extinktionswerts und eines vorab bestimmten Schwellenwerts, wobei der Schwellenwert basierend auf der Extinktion einer Lösung, in welcher die gesammelte Stuhlprobe suspendiert ist, gemessen bei der ersten Wellenlänge, bestimmt wird; und
einen Schritt des Testens der Probe zum Detektieren von fäkalem okkultem Blut, das in der Probe enthalten ist,
wobei die Probe einen Probenahmepuffer enthält und des Weiteren eine Stuhlprobe enthalten kann.

12. Verfahren zum Testen von Stuhlproben nach Anspruch 11, wobei die Probe ohne die gesammelte Stuhlprobe die Extinktion bei einem Niveau zeigt, das gleich dem vorab bestimmten Schwellenwert ist oder darunter liegt, und der Schwellenwert basierend auf der Extinktion einer Lösung bestimmt wird, in welcher die gesammelte Stuhlprobe suspendiert ist.

13. Verfahren zum Testen von Stuhlproben nach Anspruch 11 oder 12, wobei die erste Wellenlänge eine Wellenlänge ausgewählt aus dem Wellenlängenbereich von 300 nm bis 510 nm ist.

14. Verfahren zum Testen von Stuhlproben nach Anspruch 11 oder 12, wobei die erste Wellenlänge eine Wellenlänge ausgewählt aus dem Wellenlängenbereich von 300 nm bis 450 nm ist.

15. Verfahren zum Testen von Stuhlproben nach Anspruch 11 oder 12, wobei die erste Wellenlänge eine Wellenlänge ausgewählt aus dem Wellenlängenbereich von 295 nm bis 305 nm, eine Wellenlänge ausgewählt aus dem Wellenlängenbereich von 335 nm bis 345 nm, eine Wellenlänge ausgewählt aus dem Wellenlängenbereich von 375 nm bis 385 nm, eine Wellenlänge ausgewählt aus dem Wellenlängenbereich von 400 nm bis 415 nm, eine Wellenlänge ausgewählt aus dem Wellenlängenbereich von 445 nm bis 455 nm oder eine Wellenlänge ausgewählt aus dem Wellenlängenbereich von 480 nm bis 505 nm ist.

16. Verfahren zum Testen von Stuhlproben nach einem der Ansprüche 11 bis 15, wobei der Schritt des Testens der Proben Messung der Extinktion bei einer zweiten Wellenlänge durchführt, die sich von der ersten Wellenlänge unterscheidet.

17. Verfahren zum Testen von Stuhlproben nach einem der Ansprüche 11 bis 16, wobei in dem Schritt des Testens der Probe die Probe nach einem immunologischen Assay-Verfahren getestet wird.

18. Verfahren zum Testen von Stuhlproben nach Anspruch 17, wobei der immunologische Assay ein Agglutinierungs-Assay ist, der die Verwendung unlöslicher Trägerpartikel beinhaltet.

19. Verfahren zum Testen von Stuhlproben nach einem der Ansprüche 11 bis 18, wobei der Schritt des Testens der Proben Detektieren eines Markers für fäkales okkultes Blut und/oder eines Tumormarkers in der Probe umfasst.

## Revendications

1. Dispositif de test de spécimen de selles (A) comprenant une section de vérification d'échantillonnage (1-5), une section d'aspiration d'échantillon (1-4) pour collecter un échantillon à partir d'un récipient d'échantillonnage (21) et délivrer l'échantillon collecté à la section de vérification d'échantillonnage (1-5), la section de vérification d'échantillonnage étant configurée pour détecter un échantillon sans spécimen de selles collecté sur la base d'un résultat de comparaison de l'absorbance de l'échantillon mesurée à une première longueur d'onde et d'un seuil déterminé à l'avance, le seuil étant déterminé sur la base de l'absorbance d'une solution dans laquelle le spécimen de selles collecté est en suspension mesurée à la première longueur d'onde, et une section de test d'échantillon (1-6) pour détecter du sang fécal occulte contenu dans l'échantillon,
dans lequel l'échantillon contient un tampon d'échantillonnage et peut en outre contenir un spécimen de selles.

2. Dispositif de test de spécimen de selles selon la revendication 1, dans lequel l'échantillon sans le spécimen de selles collecté présente une absorbance à un niveau équivalent ou inférieur au seuil déterminé à l'avance et le seuil est déterminé sur la base de l'absorbance d'une solution dans laquelle le spécimen de selles collecté est en suspension.

3. Dispositif de test de spécimen de selles selon la revendication 1 ou 2, dans lequel la section de test d'échantillon comprend une section de mesure d'absorbance configurée pour mesurer à une deuxième longueur d'onde.

4. Dispositif de test de spécimen de selles selon l'une quelconque des revendications 1 à 3, dans lequel la première longueur d'onde est une longueur d'onde sélectionnée dans la gamme de longueurs d'onde de 300 nm à 510 nm.

5. Dispositif de test de spécimen de selles selon l'une quelconque des revendications 1 à 3, dans lequel la première longueur d'onde est une longueur d'onde sélectionnée dans la gamme de longueurs d'onde de 300 nm à 450 nm.

6. Dispositif de test de spécimen de selles selon l'une quelconque des revendications 1 à 3, dans lequel la première longueur d'onde est une longueur d'onde sélectionnée dans la gamme de longueurs d'onde de 295 nm à 305 nm, une longueur d'onde sélectionnée dans la gamme de longueurs d'onde de 335 nm à 345 nm, une longueur d'onde sélectionnée dans la gamme de longueurs d'onde de 375 nm à 385 nm, une longueur d'onde sélectionnée dans la gamme de longueurs d'onde de 400 nm à 415 nm, une longueur d'onde sélectionnée dans la gamme de longueurs d'onde de 445 nm à 455 nm, ou une longueur d'onde sélectionnée dans la gamme de longueurs d'onde de 480 nm à 505 nm.

7. Dispositif de test de spécimen de selles selon l'une quelconque des revendications 1 à 6, dans lequel la section de test d'échantillon (1-6) est configurée pour effectuer un dosage immunologique.

8. Dispositif de test de spécimen de selles selon la revendication 7, dans lequel la section de test d'échantillon (1-5) est configurée pour effectuer le dosage immunologique par un dosage d'agglutination impliquant l'utilisation de particules de support insolubles.

9. Dispositif de test de spécimen de selles selon l'une quelconque des revendications 1 à 8, dans lequel l'échantillon contenant le tampon d'échantillonnage et le spécimen de selles est une suspension de selles (15), et dans lequel la section de test d'échantillon (1-5) est configurée pour détecter un marqueur de sang fécal occulte et/ou un marqueur tumoral dans la suspension de selles (15).

10. Dispositif de test de spécimen de selles selon l'une quelconque des revendications 3, dans lequel la section de vérification d'échantillonnage (1-5) est configurée pour mesurer l'absorbance à la première longueur d'onde est également configurée pour servir de section de mesure d'absorbance à la deuxième longueur d'onde.

11. Procédé de test de spécimen de selles comprenant :
une étape de mesure d'une valeur d'absorbance d'un échantillon à une première longueur d'onde, l'échantillon ayant été collecté à partir d'un récipient d'échantillonnage (21) ;
une étape de vérification d'échantillonnage pour détecter un échantillon sans spécimen de selles collecté sur la base d'un résultat de comparaison de la valeur d'absorbance mesurée et d'un seuil déterminé à l'avance, le seuil étant déterminé sur la base de l'absorbance d'une solution dans laquelle le spécimen de selles collecté est en suspension mesurée à la première longueur d'onde ; et
une étape de test d'échantillon pour détecter du sang fécal occulte contenu dans l'échantillon,
dans lequel l'échantillon contient un tampon d'échantillonnage et peut en outre contenir un spécimen de selles.

12. Procédé de test de spécimen de selles selon la revendication 11, dans lequel l'échantillon sans le spécimen de selles collecté présente une absorbance à un niveau équivalent ou inférieur au seuil déterminé à l'avance et le seuil est déterminé sur la base de l'absorbance d'une solution dans laquelle le spécimen de selles collecté est en suspension.

13. Procédé de test de spécimen de selles selon la revendication 11 ou 12, dans lequel la première longueur d'onde est une longueur d'onde sélectionnée dans la gamme de longueurs d'onde de 300 nm à 510 nm.

14. Procédé de test de spécimen de selles selon la revendication 11 ou 12, dans lequel la première longueur d'onde est une longueur d'onde sélectionnée dans la gamme de longueurs d'onde de 300 nm à 450 nm.

15. Procédé de test de spécimen de selles selon la revendication 11 ou 12, dans lequel la première longueur d'onde est une longueur d'onde sélectionnée dans la gamme de longueurs d'onde de 295 nm à 305 nm, une longueur d'onde sélectionnée dans la gamme de longueurs d'onde de 335 nm à 345 nm, une longueur d'onde sélectionnée dans la gamme de longueurs d'onde de 375 nm à 385 nm, une longueur d'onde sélectionnée dans la gamme de longueurs d'onde de 400 nm à 415 nm, une longueur d'onde sélectionnée dans la gamme de longueurs d'onde de 445 nm à 455 nm, ou une longueur d'onde sélectionnée dans la gamme de longueurs d'onde de 480 nm à 505 nm.

16. Procédé de test de spécimen de selles selon l'une quelconque des revendications 11 à 15, dans lequel l'étape de test d'échantillon effectue une mesure de l'absorbance à une deuxième longueur d'onde qui est différente de la première longueur d'onde.

17. Procédé de test de spécimen de selles selon l'une quelconque des revendications 11 à 16, dans lequel, à l'étape de test d'échantillon, l'échantillon est testé par un procédé de dosage immunologique.

18. Procédé de test de spécimen de selles selon la revendication 17, dans lequel le dosage immunologique est un dosage d'agglutination impliquant l'utilisation de particules de support insolubles.

19. Procédé de test de spécimen de selles selon l'une quelconque des revendications 11 à 18, dans lequel l'étape de test d'échantillon comprend la détection d'un marqueur de sang fécal occulte et/ou d'un marqueur tumoral dans l'échantillon.
